# EUROPEAN PATENT APPLICATION

(11) **EP 2 647 426 A1**
(43) Date of publication of application: **09.10.2013**
(21) Application number: 12163055.2
(22) Date of filing: 03.04.2012
(51) Int. Cl.: B01J 19/00, C12Q 1/68

(54) **Replication of distributed nucleic acid molecules with preservation of their relative distribution through hybridization-based binding**

(71) Applicant: Max-Planck-Gesellschaft zur Förderung der Wissenschaften e.V., 80539 München (DE)
(72) Inventor: Soldatov, Aleksey, Dr., 14195 Berlin (DE); Borodina, Tatiana, Dr., 14195 Berlin (DE); Lehrach, Hans, Prof., 14129 Berlin (DE)
(74) Representative: Arth, Hans-Lothar

(57) **Abstract**

The present invention provides methods for preserving information about original spatial distribution of nucleic acid molecules transferred from a sample to another surface or into solution.

SEQUENCE LISTING

<110>  Max Planck Gesellschaft zur Förderung der Wissenschaften
       e.V.

<120>  Replication of distributed nucleic acid molecules with
       preservation of their relative distribution through
       hybridization-based binding

<130>  GAR-P03414EP

<160>  3

<170>  PatentIn version 3.3

<210>  1
<211>  30
<212>  DNA
<213>  Artificial

<220>
<223>  Oligonucleotides for immobilization

<400>  1
tttttttttt aatgatacgg cgaccaccga                                        30


<210>  2
<211>  31
<212>  DNA
<213>  Artificial

<220>
<223>  Oligonucleotides for immobilization

<400>  2
tttttttttt caagcagaag acggcatacg a                                      31


<210>  3
<211>  32
<212>  DNA
<213>  Artificial

<220>
<223>  Hybridization probe

<400>  3
agagtgtaga tctcggtggt cgccgtatca tt                                     32

## Description

In the present invention a replica of distributed nucleic acid molecules is created by hybridization of nucleic acids located on a surface or within a layer of a sample to a target surface by hybridization to complementary sequences. Target surface may be for example covered with oligonucleotides, complementary to the nucleic acids on the sample or hybridization may occur through adapter oligonucleotides complementary both to the oligos on the target surface and to the nucleic acid molecules on the sample.

### Background of the invention

Biological processes are spatially organized. They rely upon the interplay of many different components forming an intricate structure of cells, tissues and organisms. Molecules participating in these processes have a certain spatial distribution. Understanding the biological processes is critically dependent on a detailed knowledge of this distribution.

Objects with two-dimensional distribution of nucleic acid molecules, for example tissue sections, are widely studied. There exist methods for nucleic acid analysis in tissue sections, for example *in situ* hybridization or *in situ* PCR. However, not all molecular biology methods are applicable when working with tissue sections.

Two-dimensional tissue sections are convenient objects to study distribution of molecules. Several sequential sections restore a 3D spatial location of molecules. However, many molecular biology methods, for example sequencing, cannot be performed directly in tissue sections. It would be advantageous to be able to transfer molecules from the tissue section to another surface or into solution, where appropriate methods of analysis could be performed. However, such transfer raises questions of keeping information about initial distribution of the nucleic acid molecules.

Replication of 2D distributed objects (nucleic acid molecules, cells) has been long used in molecular biology. Main purposes are to perform analysis which is not possible with original sample and (ii) multiplying 2D sample for several analyses.

Southern and Northern methods are known, wherein nucleic acid molecules are transferred from gel to membrane. Membrane allows analyzing transferred molecules by hybridization preserving the relative distribution they had in gel. Replica of DNA of library clones on membranes is used to search for particular clones using hybridization. Replication of bacterial colonies to other plates allows analyzing in parallel, for example, their resistance to several antibiotics.

In the last decade, two methods were suggested for multiplying nucleic acid arrays by replication. In one approach nucleic acid array features are first amplified on the array, then the array with amplified features is brought into tight contact with transfer support, to which parts of amplified molecules are transferred and get covalently attached (U.S. Patent 7,785,790). In the nanostamping approach nucleic acid molecules hybridised to sample surface are brought into direct contact with capturing groups on the target surface. Chemical binding with the target surface is stronger than hybridization and after separating surfaces, nucleic acid molecules remain on the target surface (U.S. Patent 7,862,849).

The general principle of replication is bringing into contact a surface with 2D distributed nucleic acid molecules with a target surface, to which they are transferred by diffusion or direct contact. So far nucleic acid molecules have been transferred to surfaces were they were captured either physically (stuck in gel) or by chemical bonds (covalent, ion exchange, affinity) involving certain reactive groups on the nucleic acid molecules and on the target surface, but not involving the nucleotide sequence of the molecules.

Objective of the present invention is to provide a method capable of preserving the information about spatial distribution of nucleic acid molecules transferred from a surface to another surface.

This objective is solved by the present methods as shown below. Further preferred embodiments of the present invention are disclosed in the dependent claims, the description, the figures and the examples.

Surprisingly it was found that methods according to the present invention allow the transfer of nucleic acid molecules to another surface preserving information about their original positions.

### Description of the invention

The present invention is directed to methods for preserving information about original spatial distribution of nucleic acid molecules transferred from a surface to another surface or into solution.

This is accomplished by the use of hybridization of nucleic acids for creating replicas of nucleic acids molecules or molecular complexes containing nucleic acid molecules located either on a surface or within a layer, wherein said creating replicas is obtaining on a target surface the relative distribution of nucleic acid molecules or molecular complexes containing nucleic acid molecules resembling the original distribution.

Hybridization as a way to capture nucleic acid molecules makes the replication highly selective, since only nucleic acid molecules having complementary sequences will be hold on the target surface. Besides, hybridization is a controllable process and allows regulation of the time of replication and, consequently, the number of transferred nucleic acid molecules. The method does not require direct contact of 2D distributed nucleic acid molecules to binding sites on the target surface. This means that (i) the transfer may be performed between large solid surfaces, which can't form uniform tight contact and (ii) the method may be applied to transfer nucleic acid molecules from 3D samples to the target surface.

The term "replica" as used herein refers to a copy of the distribution of nucleic acids with preservation of their original distribution to a target surface by hybridization. The target surface with the transferred nucleic acids held by hybridization with preservation of their original distribution is the created replica. Thus, replica is obtaining on a target surface the relative distribution of nucleic acid molecules or molecular complexes containing nucleic acid molecules resembling the original distribution.

The replication method according to the invention comprises the following steps:
a) providing sample with nucleic acid molecules located either on a surface or within a layer;
b) providing target surface with nucleic acid molecules, capable to hybridization-based binding to nucleic acid molecules from (a);
c) (option 1) if nucleic acid molecules are not attached to the sample, providing conditions to minimize shift of molecules from the original positions;
d) (option 2) if nucleic acid molecules are attached to the sample, providing conditions for gradual releasing of nucleic acid molecules;
e) assembling sample with nucleic acid molecules from (a) against the target surface from (b) with solution in between, such that nucleic acid molecules from the sample can reach target surface by diffusion through solution;
f) (option 3) if nucleic acid molecules are attached to the sample, providing conditions for releasing nucleic acid molecules from the original positions in the sample,
g) providing conditions for diffusion of nucleic acid molecules from the sample to the target surface and hybridization-based binding of nucleic acid molecules from the sample to the nucleic acid molecules on the target surface;
h) (optional) providing conditions for slowing down the formation of new hybrids of nucleic acid molecules;
i) disassembling the sample (a) and target surface (b).

The nucleic acid molecules can be either located on the surface of the sample or within a layer of the sample. Preferably, the nucleic acid molecules located on a surface or within the layer are distributed on a nucleic acid array or protein array, and wherein said nucleic acid molecules distributed within a layer are distributed in a gel layer, in tissue section, in cell or tissue array or in block of tissue. For example, the nucleic acids can be contained in a gel and can be mobilized out of the gel to the surface of the gel. Alternatively, the nucleic acids can be provided on a glass slide.

The sample with nucleic acid molecules also comprises nucleic acids that are hybridized to the nucleic acids on the sample. This means that nucleic acids could be distributed on the sample and to this nucleic acids further nucleic acids are hybridized. Thus providing a sample with nucleic acids molecules located either on a surface or within a layer also includes hybridization products of nucleic acids. Consequently, the term nucleic acids also comprise hybridization products of nucleic acids.

The target surface comprises a plurality of at least one type of oligonucleotides attached to the target surface. The target surface can be of any texture. The target surface should be covered with oligonucleotides, at least in the area to which the transfer is performed. Transferred nucleic acid molecules hybridize preferably directly to the oligonucleotides immobilized on the target surface.

A lot of ways are known in the prior art for preparation of surfaces covered with oligonucleotides, e.g. spotting, on-surface synthesis, attaching to beads, fixation in gel, etc.

The nucleic acids may be either attached or not attached to the sample. If the nucleic acids are not attached to the sample it is preferred to apply conditions to minimize the shift of nucleic acid molecules from their respective original positions. Such conditions could be the decrease of temperature. Preferably, the temperature is decreased below 24°C, preferably 20°C, more preferably 16°C, preferably 12°C, even more preferred 8°C, and more preferred 4°C.

On the other hand if the nucleic acids are attached to the sample it may be advisable to apply conditions, wherein gradual release of the nucleic acids in the sample occurs. The nucleic acids may be attached to the sample, for example by hybridization to complementary sequences covalently bound to the sample, or through cleavable groups.

In one embodiment the nucleic acid molecules in the sample are held on the original positions by chemical- or enzyme- sensitive binding. Thus before assembly of the sample with the target surface conditions can be applied, wherein gradual release of the nucleic acid molecules from the sample occurs. Said conditions for gradual release of nucleic acid molecules from the sample may be the cleavage agent which acts slow enough to ignore those molecules which change the position before assembling sample against the target surface

In one embodiment said low activity of the cleavage agent is provided by decreasing concentration of the said agent or by providing reaction conditions decreasing the activity of the said agent.

Diffusion of nucleic acid molecules within the sample may be physically hindered by surrounding matrix, for example agarose or acrylamide gel. In this case diffusion exists but it is very slow: the time of appearing of free molecules on the surface of the sample is much longer than the time of assembling sample/target surface sandwich. It is possible to assemble a sandwich and wait till nucleic acid molecules diffuse enough to reach the target surface. It might be possible to speed up the diffusion by raising the temperature of the sandwich. Nucleic acid molecules may be just physically stuck within the sample, for example in gel after gel electrophoresis.

The sample with nucleic acid molecules and the target surface are assembled under conditions where nucleic acid molecules do not go off their positions on the sample surface. Such conditions can comprise e.g. low temperature, a filter or net between the surfaces, enzymes and/or chemical substances preventing detachment at this stage or vice versa the lack of such enzymes and/or chemical substances needed for detachment.

Preferably the sample with nucleic acid molecules and the target surface are assembled under "wet" conditions meaning that the sample and target surface are surrounded by solution, i.e. liquid and/or that liquid is between both surfaces. Both surfaces are arranged such that both surfaces come into contact with each other in a sandwich-like configuration. A thin liquid film can exist between both surfaces. The liquid between the surfaces and/or around the assembled sandwich-like configuration can comprise enzymes and/or chemical substances needed e.g. for detachment. If a filter or net between the surfaces is used during assembly, such a net would prevent direct contact of the surfaces.

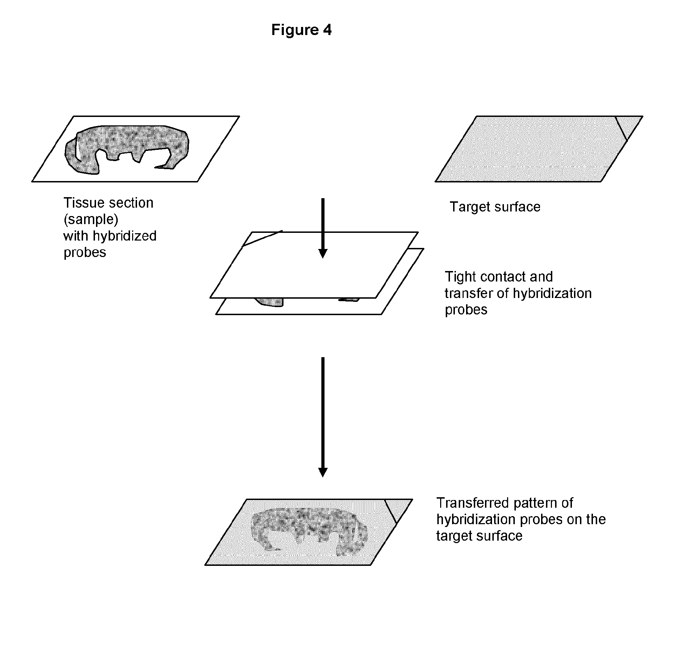

The surfaces in the sandwich-like configuration shall be tightly pressed to each other to make the distance between the surfaces so that the distance between both surfaces is so small that no blurring of the distribution pattern occurs. Assembling such sandwich-like configurations is performed as shown in Fig.4 and is well known to the skilled artisan and corresponds *mutatis mutandis* to the procedures known from e.g. western/northern blotting. Surface assembly would be done preferably at room or lower temperature, so that the nucleic acid molecules do not go off the sample surface. Generally, the inventive method does not require a direct contact between the nucleic acids distributed on the sample and the oligonucleotides on the target surface. This means that transfer may be performed between large solid surfaces, which can't form uniform tight contact.

The terms "sandwich-like configuration" or "assembly" both refer to the configuration that the sample and the target surface are brought into contact with each other.

The incubation time is dependent from many variables, such as accessibility of the nucleic acids on the sample, incubation temperature and other factors. Generally, incubation time should be long enough to allow sufficient hybridization, but still short enough to prevent e.g. unspecific binding. Under aspects of process economy, the incubation time should be chosen to be as short as possible. The skilled artisan can determine the optimal incubation time with minimum routine experimentation.

Detachment conditions (certain temperature, light, solution) may be applied to the assembly of the sample with distributed nucleic acid molecules and the target surface. Temperature may be applied to release nucleic acid molecules if the binding to the sample is temperature-sensitive. Thus, in one embodiment the condition for releasing the nucleic acid molecules from the original positions in the sample occurs by increasing the temperature.

In another embodiment the nucleic acid molecules are held on the original positions in the sample by temperature-sensitive binding by hybridization or through thermolabile covalent bonds, abasic site or formaldehyde linkage. Detachment can also occur by providing a thermoactivated cleavage agent, enzyme or chemical reagent in the solution between the sample and the target surface.

Hence, in another embodiment the condition for releasing the nucleic acid molecules from the original positions in the sample is changing the solution between the sample and the target surface.

The possibility to change solution in the contact area in the assembly substantially increases the variants of nucleic acid molecules attachment to the sample, and consequently, types of samples. If nucleic acid molecules are attached to the nucleic acid sample by hybridization to a complementary sequence, duplex may be denatured by changing the pH or ionic strength of the solution, or changing the solution to the one decreasing the denaturation temperature (like formamide). Nucleic acid molecules may be attached through some cleavable group. The cleavage agent (e.g. enzyme or chemical substance) may be delivered after the sandwich assembly.

In yet another embodiment the nucleic acid molecules are held on the original positions in the sample by hybridization and the new solution destabilizes hybridization by changing pH or ionic strength of the solution or decreasing the melting temperature of the duplex like formamide, or the nucleic acid molecules are held on the original positions in the sample by chemical- or enzyme- sensitive binding and said new solution contains a cleavage agent, and wherein either the sample or the target surface or both are permeable for the said solution and during changing of the solution the assembly remains intact. Thus, only the solution is changed but the integrity of the assembly is not changed, i.e. the assembly of the sample and target surface is not disassembled.

If nucleic acid molecules are attached to the sample by hybridization to a complementary sequence, duplexes may be denatured by heating the assembly. Nucleic acid molecules may be covalently attached to the sample through thermolabile bonds like abasic site or formaldehyde linkages. In such cases heating would destroy the binding. Binding may also be organized through enzymatically or chemically cleavable site, where cleavage enzyme or chemical reagent should be thermoactivated. Cleavage agent should then be present in the solution, but during assembling the sandwich it should not act (e.g. to prevent working of an enzyme sandwich may be assembled at low temperature) or should act slowly (e.g. low concentration, inappropriate temperature). In one embodiment light may be applied to release molecules attached to the sample through photocleavable groups. In this case either the nucleic acid on the sample or the target surface or both should be translucent for the light of the required wavelength. Sandwich should be assembled without the activating light.

Under certain conditions it may be necessary to wash the sample after incubation. Washing can be performed with known washing buffers, such as PBS or any other washing buffer known to the skilled artisan. Care should be taken not to use washing buffer, which are able to disrupt the bonding between the hybridized nucleic acid molecules and their complementary sequences.

An "oligonucleotide" as used herein is a short nucleic acid polymer, typically with fifty or fewer bases. Although for the purposes the present invention, the oligonucleotides can have more or less nucleic acids.

Before separating the surfaces, it may be necessary to decrease the temperature close to 0°C. At low temperature hybridization speed becomes low, which prevents attaching of nucleic acids to the wrong places on the target surface when the sandwich-like configuration is disturbed. Optionally, washing of the target surface may be performed at low temperature. Thus, in one embodiment before disassembling the sample and the target surface slowing down formation of new hybrids of nucleic acid molecules is done by decreasing the temperature of the assembly.

In one embodiment a plurality of adapter oligonucleotides is provided. The adapter oligonucleotides are complementary both to the nucleic acid molecules from the sample and to the nucleic acid molecules on the target surface. These adapter oligonucleotides are characterized by at least two regions, wherein one region is at least partially complementary to a nucleic acid on the sample and another region is at least partially complementary to the oligonucleotides attached to the target surface. In this embodiment the nucleic acids do not hybridize directly to the at least one type of oligonucleotides on the target surface but said hybridization-based binding occurs through adapter oligonucleotides which are complementary both to the nucleic acid molecules from the sample and to the nucleic acid molecules on the target surface.

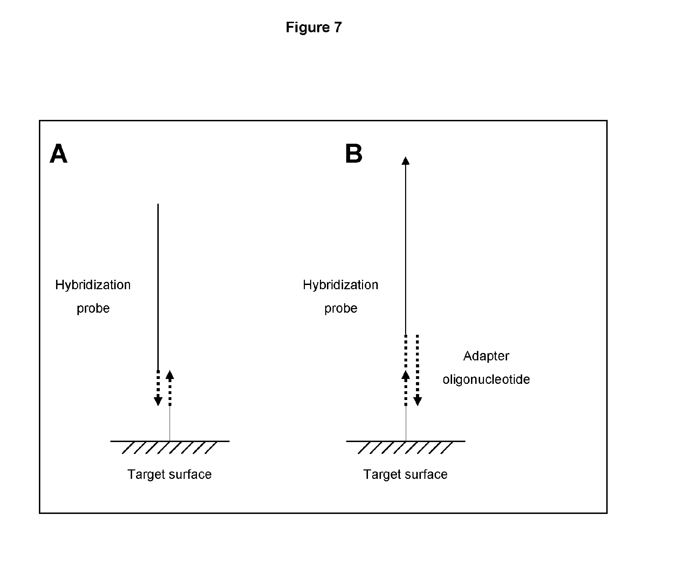

The general mechanism is a shown in Fig.7 (B) in comparison to direct hybridization of the nucleic acids to the target surface as shown in Fig.7 (A).The use of adapter oligonucleotides allows to use the same target surfaces for hybridization probes with different regions responsible for binding to the target surface.

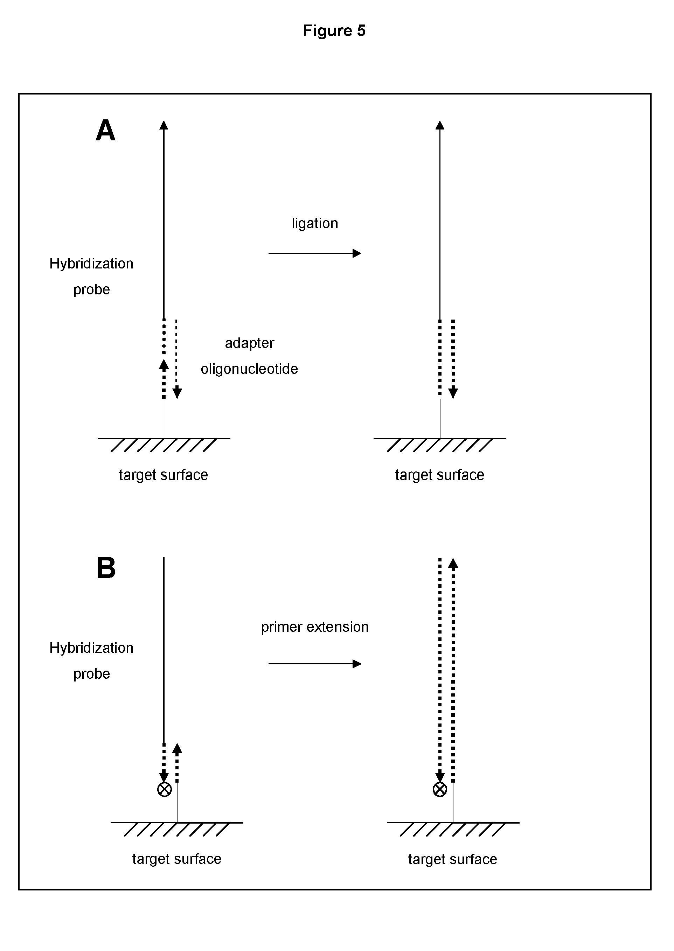

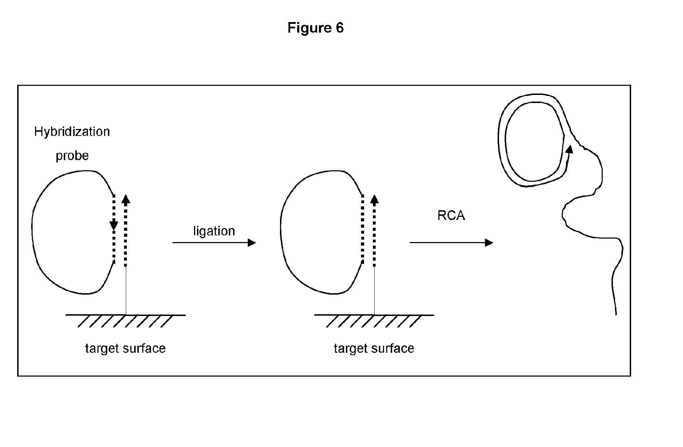

After the nucleic acids from the sample have been transferred to the target surface enzymatic reactions may be performed with the replica on the target surface, wherein said enzymatic reactions include primer extension, ligation, rolling circle amplification, in situ PCR amplification, bridge PCR amplification, sequencing, restriction (see Fig.5 and 6).

In yet another embodiment the nucleic acid molecules in the sample or nucleic acid molecules on the target surface contain known sequences, which (optionally) get inserted in the nucleic acid molecules from the target surface or nucleic acid molecules from the sample by primer extension or ligation reactions and said known sequences are further used for analysis of replicas, wherein said analysis may be performed on the target surface or in solution.

In another embodiment the said known sequences serve to distinguish the samples, target surfaces, replication experiments, wherein said known sequences are different between the samples, target surfaces, replication experiments or to determine the position of nucleic acid molecules on the target surface or in the sample and wherein said known sequences are different in different regions of the sample or of the target.

Oligonucleotides on the target surface may contain besides the regions for hybridization-based binding of nucleic acid molecules from the sample, sequences for labeling the transferred nucleic acid molecules. Such sequences get attached to the transferred nucleic acid molecules or their derivatives (extention, ligation products) after replication by ligation or primer extension. In the following analysis of the replicated molecules or their derivatives, for example by sequencing or hybridization, the labeling sequence would reveal to which oligonucleotide a certain replicated molecule was bound.

Labeling sequences may be used for position coding of the transferred nucleic acid molecules. For example, target surface may be divided into a number of small regions (code regions), oligonucleotides in each region containing unique nucleic acid codes - a 4-100nt nucleic acid sequence. Coding target surface may be used for position coding of transferred nucleic acid molecules: in each code region a different nucleic acid code will be added to the nucleic acid molecules. Adding may be performed by for example ligation, primer extension in appropriate conditions. By adding position-specific codes, information about surface coordinates of nucleic acid molecules is recorded in the sequences of nucleic acid codes. It is then possible to remove the coded replicated nucleic acid molecules from coding surface into solution. In the course of further analysis reading of the codes gives information about original positions of nucleic acid molecules.

In these embodiments the hybridization probes are transferred to a target surface with preformed coded regions - thus, hereinafter named coding surface - and coding oligonucleotides already distributed on the coding surface.

The general procedure is that prior to transfer of the nucleic acids from the sample to the coding surface, so called code regions are created on the coding surface. Thus the coding surface is subdivided in any number of code regions, the number of code regions being dependent on the desired resolution. The code regions can be created physically, by applying e.g. a filter or net on the original surface, wherein each "hole" in this net or filter would represent one code region. It is also possible to use beads with coding oligonucleotides attached to them, wherein each bead would correspond to one coding region. However, it is also possible that the code regions are not created physically but only imaginary code regions are created. This could be realized by e.g. registering the coordinates of each code region on the sample. The coding surface comprises a plurality of coding oligonucleotides attached to the target surface. As long as the coding surface can bind oligonucleotides to its surface, the coding surface can be of any texture. The coding surface consists of code regions in each code region coding oligonucleotides have a different nucleotide code. The more precise localization of transcripts is required, the smaller code regions should be used. The more code regions should be on the coding surface - the longer code regions are required to have a unique code in each code region.

Such coding surface may be prepared for example by spotting nucleic acid codes, by making layer of beads with nucleic acid codes, by synthesizing nucleic acid codes directly on the surface.

The transferred nucleic acids would be coded using primer extension reaction: depending on the unique nucleic acid sequence in the coding oligonucleotides, nucleic acids will be extended with a certain unique sequence. The primer extension mix would contain nucleotides and polymerase in an appropriate buffer. Care has to be taken that during primer extension reaction, the nucleic acids do not go off their locations. Therefore, extension should be performed at temperatures below annealing temperature of the nucleic acids.

The result of the extension would be a double-stranded molecule, in which both stands have flanking regions required for sequencing and unique nucleic acid sequence from the coding oligonucleotides, required for revealing the original position on the original surface. The coded extension products can be removed from the double-stranded molecule by different methods. In one embodiment the coding surface is rinsed high-temperature (∼95°C) solution. At high temperature, the double strands will be denatured and the non-covalently attached stands go into solution. Also high temperature inactivates the enzyme used for primer extension, so that no primer extension is possible in the solution.

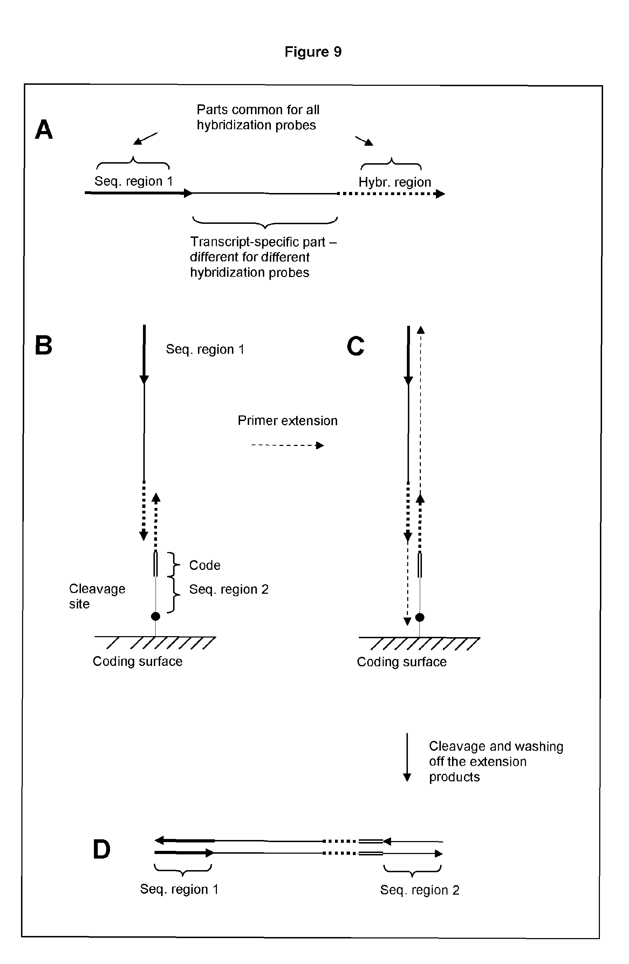

In another embodiment, the coding oligonucleotides on the coding surface would further comprise a cleavable group. Due to this cleavable group, the whole double strand can be removed from the coding surface after destroying the cleavable group. The double strand (see Fig.9d) may be further amplified and then sequenced.

It should be taken into consideration, that during transfer of nucleic acid molecules to the coding surface and during adding of nucleic acid codes to the nucleic acid molecules, nucleic acid codes should stay within the coding regions. Depending on the way of attachment of coded replicated nucleic acid molecules to the coding surface, nucleic acid molecules may be released independently from non-used nucleic acid codes or together with them. For example, when coded nucleic acid molecules are attached to the coding surface by hybridization, and nucleic acid codes are covalently attached, nucleic acid molecules may be released from the surface by denaturizing conditions, and nucleic acid codes will remain on the surface. When both nucleic acid codes and coded replicated nucleic acid molecules are attached to the coding surface in the same way, they will be released together. In the latter case nucleic acid codes either remain in the mixture with coded nucleic acid molecules if they do not interfere with further operations, or they would be removed, for example by size selection.

The present invention is also directed to a coding surface with a plurality of coding regions, wherein the coding surface is covered with a plurality of coding oligonucleotides, wherein the coding oligonucleotides are characterized by a 3' part common to all coding oligonucleotides, and an individual nucleotide sequence of 4 - 100 nucleotides, characterized in that each coding region is covered only with coding oligonucleotides with the same individual nucleotide sequence of 4 - 100 nucleotides.

### Description of Figures

- Fig. 1:: Cy3-labeled oligonucleotides #003 was hybridized to the slides #1a (with oligonucleotides #001 deposited to form figure "1") and to the slide #2 (with oligonucleotides #002 deposited to form figure "2").
- Fig. 2:: Transfer of Cy3-labeled oligonucleotide #003 hybridized to the slide #1 b_hybr to the slide #3. The surface of the target slide #3 is covered with covalently immobilised oligonucleotides #001 (grey area), which is complementary to #003.

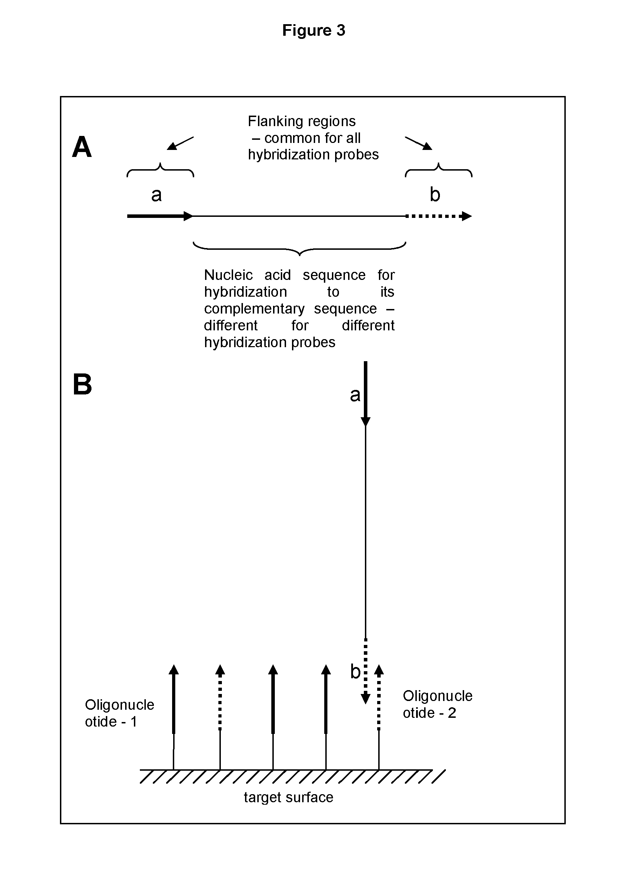

- Fig. 3:: (A) Structure of hybridization probe with two flanking regions suitable both for (i) hybridization to nucleic acids on sample surface and for (ii) second generation sequencing. (B) Scheme of the probe hybridization to the target surface. The target surface is covered with attached oligonucleotides - 1 and - 2. Oligonucleotides - 2 hybridizes with region b of the hybridization probe, providing attachment to the target surface. Oligonucleotides - 1 hybridizes with sequence complementary to region a of the hybridization probe, making possible the on-surface amplification and second generation sequencing.
- Fig. 4:: Scheme of transfer of hybridization probes from sample surface to the target surface
- Fig. 5:: Examples of enzymatic reactions which may be performed with hybridization probes on the target surface. Hybridization probes can be sequenced on the target surface, using the oligonucleotides on the target surface to start sequencing-by-synthesis. Hybridization probes may be amplified, for example by rolling circle amplification (RCA), in *situ* PCR or bridge PCR.
- Fig. 6:: Scheme of rolling circle amplification of the hybridization probe. Hybridization probe is circularised and amplified using oligonucleotides on the target surface first as a template for ligation and then as a primer for amplification.
- Fig. 7:: Examples of attaching hybridization probes on the target surface by hybridization. (A) Hybridization of hybridization probes directly to the oligonucleotides attached to the target surface. (B) Binding to the target surface by hybridization to adapter oligonucleotides.
- Fig. 8:: (A) Structure of hybridization probe with one flanking region suitable both for (i) hybridization to nucleic acids on sample surface and for (ii) second generation sequencing. (B) Scheme of the probe hybridization to the target surface. The target surface is covered with attached oligonucleotides. The oligonucleotides hybridize with region "a" of the hybridization probe, providing attachment to the target surface. Second generation sequencing can be performed without amplification according to true single molecule sequencing tSMS™ (Helicos).
- Fig. 9:: (A) Structure of hybridization probe suitable for (i) hybridization to complementary sequences on sample surface, (ii) hybridization to the coding surface and having seq. region 1 necessary for second generation sequencing. (B-D) Scheme of adding a code to hybridization probes using primer extension. Oligonucleotides attached to the coding surface have 3' end necessary for hybridization to the hybridization probes transferred from sample surface, code region to mark the location of the hybridization probes and seq. region 2 required for second generation sequences (B). To add codes and seq. region 2 to hybridization probes, extension reaction is performed (C). Resulting double-stranded molecules suitable for second generation sequencing are cleaved from the surface (D) and combined in solution.
- Fig. 10:: Scheme of position coding involving coding surface. Hybridization probes are transferred to a coding surface

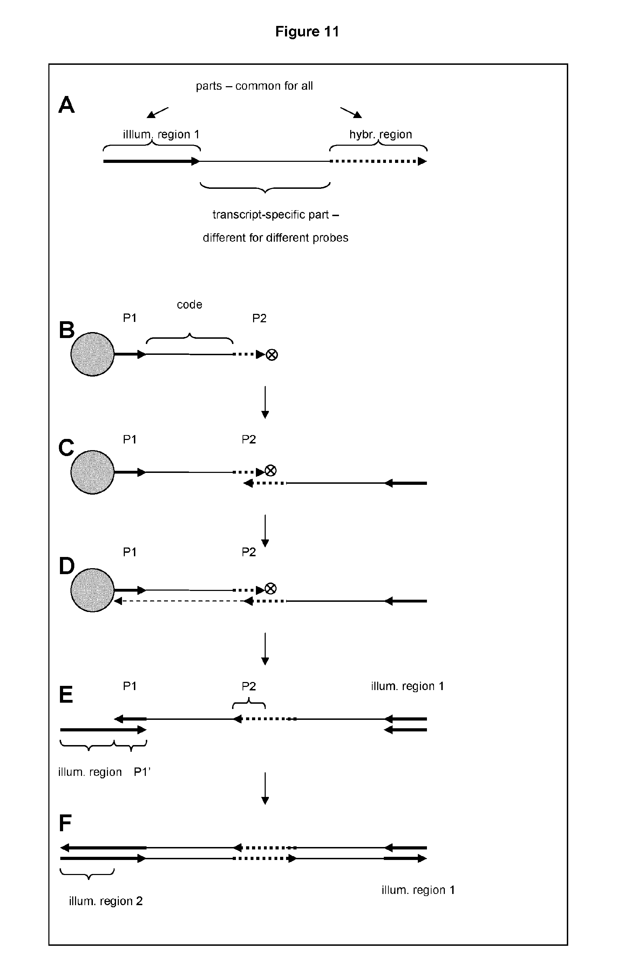

- Fig. 11:: (A) Structure of hybridization probe suitable for (i) hybridization to transcripts in tissue section, (ii) hybridization to the SOLiD P1 region of sequencing templates and having illum. region 1 necessary for Illumina SGS. (B) Structure if the SOLiD sequencing template attached to the bead. (C-F) Scheme of adding a code to hybridization probes using primer extension. Hybridization probes transferred from tissue section slide hybridize to the P2 region (C). Hybridization probes are extended (D). Internal sequence of the sequencing template which marks the position of the bead on the SOLiD flowcell is now added to the hybridization probe sequence, thus marking the position of the transfer nd of original transcript in tissue section. To introduce illum. region 2 necessary for Illumina SGS, coded hybridization probes are PCR amplified. One of PCR primers has a P1-complementary 3' end and illum. region 2 5' tail; another primer correspond to illum. region 1 (E). Resulting double-stranded molecules are suitable for Illumina SGS.
- Fig. 12:: Position coding involving sequenced SOLiD flowcell as a coding surface. Hybridization probes are transferred to a coding surface covered with beads. Each bead is characterised by a specific coding sequence.
- Fig. 13:: Selective replication of full-length oligonucleotides by hybridization to the sequences complementary to the 5' end of oligonucleotides. (A) Full-length oligonucleotides have the same sequence on 5' end. (B) Full-length oligonucleotides in each feature of the oligonucleotide array have a different sequence on 5' end. In this case target surface is organized as an array of features with oligonucleotides complementary to full-length oligos in the corresponding features of oligonucleotide synthesis surface.

### Examples

### Example 1: Oligonucleotide transfer

### Consumables

● Epoxy-modified glass slides: Nexterion Epoxysilane 2-D surface Slide E kit (Schott, #1066643)
● Hybridization chambers: Secure Seal (Grace bio-labs, #SA500)
● Oligonucleotides:
   ■ SH-modified oligonucleotides for immobilization on the epoxy slides:
      #001 5' SH-TTTTTTTTTTAATGATACGGCGACCACCGA 3' (SEQ ID NO:1)
      #002 5' SH-TTTTTTTTTTCAAGCAGAAGACGGCATACGA 3' (SEQ ID NO:2)

The unique sequences correspond to the sequences of oligonucleotides immobilized on the Illumina sequencing flowcells;
■ Cy3-labeled fluorescent hybridization probe:
   #003 5' Cy3-AGAGTGTAGATCTCGGTGGTCGCCGTATCATT 3' (SEQ ID NO:3)

Partly complementary to oligonucleotides #001, complementary sequence is underlined.

### Slides prepared

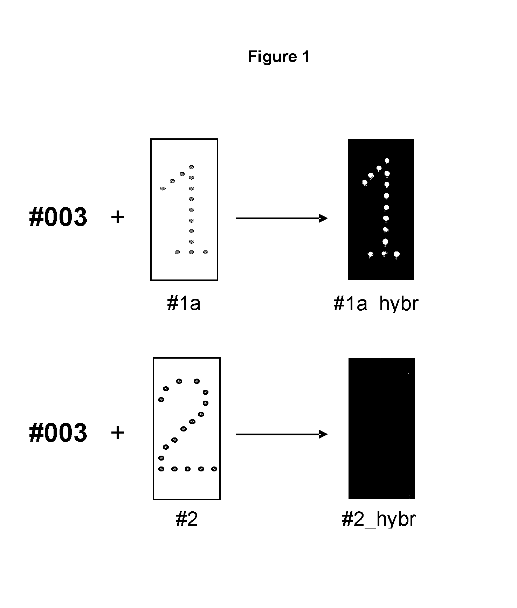

SH-modified oligo #001 was immobilized on five epoxy slides: on three slides - in a recognizable pattern and on the other two - over the whole surface. SH-modified oligo #002 was immobilized on three epoxy slides: on one slide - in a recognizable pattern and on the other two - over the whole surface.
1. 40 µM SH-modified oligos were diluted to 20 µM by adding the equal volume of the 2x Nexterion Spot solution.
2. Oligo solutions were deposited on slides:
   ● Slides #1a, #1b and #1c: 1 µl drops of oligo #001 were deposited to form a figure "1" (see figure 1, #1a)
   ● Slide #2: 1 µl drops of oligo #002 were deposited to form a figure "2" (see figure 1, #2)
   ● Slides #3&4: were laid upon each other with 3 mm wide and 0.2 mm thick spacers along the long sides and oligo #001 was pipetted to fill the space between the two slides;
   ● Slides #5&6: were laid upon each other with 3 mm wide and 0.2 mm thick spacers along the long sides and oligo #002 was pipette to fill the space between the two slides.

Further all slides (#1a, b, c - 6) were handled the same way.
3. Slides with deposited oligonucleotides were incubated in a humidity chamber at room temperature for 30 min to ensure quantitative immobilization.
4. Slides were washed at room temperature:
   ● for 5 min in 0.1 % Triton® X-1 00;
   ● two times for 2 min in 1 mM HCl solution;
   ● for 10 min in 100 mM KCI solution;
   ● for 1 min in bidistilled water.
5. Blocking was performed:
   ● incubating for 15 min in 1x Nexterion Blocking Solution at 50°C;
   ● rinsing for 1 min in bidistilled water at room temperature.
6. Slides with immobilized oligonucleotides were dried under nitrogen stream and stored in dry atmosphere in an excicator.

### Hybridization of Cy3-labeled oligonucleotides to the slides #1a, b, c and #2.

1. Cy3-labeled oligonucleotides #003 solution was prepared: 10 nM oligo in 90 % Nexterion Hybridization buffer.
2. Hybridization chambers were placed over the areas with spots on slides #1a, #1b, #1c and #2, the labelled oligonucleotides solution was added to the chamber.
3. Slides were incubated for 1 hour at 42°C in the PCR machine with glass slides adapter.
4. Hybridization chambers were removed and slides were washed at room temperature:
   ● for 10 min in (2x SSC, 0.2 % SDS);
   ● for 10min in 2x SSC;
   ● for 10min in 0.2x SSC.
5. Slides #1b and #1c with hybridized Cy-3 labeled oligo (#1b_hybr and #1c_hybr) were left in 0.2x SSC at room temperature for ∼ 1 hour, till the transfer experiment was performed.
6. Slides #1a and #2 with hybridized Cy-3 labeled oligo (#1a_hybr and #2_hybr) were dried under nitrogen stream and scanned on the Affymetrix 428 Array Scanner.

On slide #1a_hybr a fluorescent pattern of the figure "1" was obtained (see Fig. 1). No fluorescent signal was observed on the slide #2_hybr.

### Transfer of the Cy-labeled oligonucleotide #003 hybridized to slides #1b_hybr and #1cc_hybr to the slides #3 and #5

Cy3-labeled oligonucleotides #003 hybridized to the slide #1b_hybr was transferred to the slide #3 covered with oligo #001, complementary to #003. Cy-3 labeled oligonucleotide #003 hybridized to the slide #1 c_hybr was transferred to the slide #5 covered with oligo #002, not complementary to #003.
1. ∼25 µl of Nexterion Hybridization bhuffer was pipetted on the oligo covered surfaces of slides #3 and #5, to which the Cy-3 labeled oligo had to be transferred.
2. Slides #1b_hybr and #1c_hybr with the hybridized fluorescent oligonucleotide #003 were placed over the drop of Nexterion Hybridization buffer on slides #3 and #5 respectively.
3. The sandwiches of slides #1b_hybr / #3 and #1c_hybr / #5 were placed in separate plastic bags.
4. The slides in both sandwiches were pressed tightly to each other with paper clips to let the hybridization buffer squeeze out into the bag.
5. Bags with slide sandwiches were placed in a beaker with boiling water for 3min.
6. Bags were transferred to a beaker with 42°C water for 15 min.
7. Bags were transferred to room temperature; sandwiches were taken out and disassembled. All slides were washed, blocked, dried out and scanned as described in the "Hybridization" section.

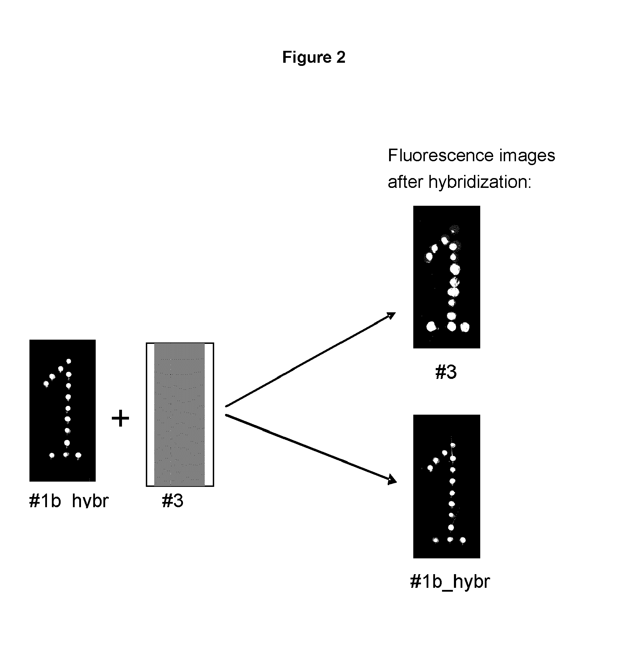

On slide #3a mirror replica of the fluorescent pattern of the figure "1" from slide #1b_hybr was obtained. Thus, the Cy-3 labeled oligo #003 hybridized to the slide #1b_hybr has been transferred to the surface of slide #3 (see Fig. 2). No transfer of oligo #003 from slide #1c_hybr to the slide #5 was observed.

### Example 2: Analysis of transcripts in tissue sections

Two-dimensional tissue sections are convenient objects to study distribution of molecules. Several sequential sections restore a 3D spatial location of molecules.

Currently, transcripts in tissue sections are analyzed by in situ hybridization. Main restriction of this approach is the limited number of transcripts which it is possible to analyze simultaneously. The reason is that it is impossible to select considerable number of distinguishable labels for hybridization probes.

However many molecular biology methods, for example, sequencing, cannot be performed directly in tissue sections. It would be advantageous to be able to transfer molecules from the tissue section to another surface or into solution, where appropriate methods of analysis could be performed.

In this example it is described how transcripts in tissue sections may be analyzed by sequencing and ex situ hybridization.

In the second generation sequencing (SGS) platforms sequencing is performed on the surface of the special flowcell for millions of templates in parallel. 2D flowcell surface is similar to the slide with tissue section. Sequencing cannot be performed directly in the tissue section. However using the method of the invention it is possible to transfer the transcripts (hybridization probes, primer extension products) from tissue section to the surface of sequencing flowcell preserving the distribution pattern.

The experiment is conducted according the following flow chart:

preparation of hybridization probes suitable both for hybridization to
the nucleic acids on the sample surface and with flanking regions for
second generation sequencing and hybridization to the
oligonucleotides on the target surface

in situ hybridization of the sample, washing off the unhybridized

transfer of hybridized probes to the target surface (scheme is on
Figure 4):
- surface sandwich assembly
- denaturation of hybridized probes
- hybridization to the target surface
- stopping the hybridization to the target
- disassembling of the sandwich
- washing off the unhybridized probes

surface amplification and sequencing on the target surface

analysis of sequencing results: identification of transcripts and their
relative positions on the original surface

Hybridization probes would have the structure as shown on Fig.3A. Middle parts of probes are for hybridization to transcripts in tissue section. Flanking regions a and b are common for all probes and are required both for hybridization and sequencing on the SGS flowcell surface (Fig.3B).

Hybridization probes may be selected to target from single to thousands of transcripts. They may be synthesized artificially or prepared from a sequencing library.

To prevent unspecific hybridization of common parts of the hybridization probes in tissue section it is possible to reversibly block them with complementary oligonucleotides. These oligonucleotides should be removed before transfer of hybridization probes to the SGS flowcell surface.

Tissue section slide and SGS surface would be brought into tight contact, possibly with a net in between (see Fig.4). The distance between surfaces (or the mesh size if a net is used) should be smaller than acceptable blurring of the distribution pattern. A net would also prevent direct contact of the tissue section and SGS surface.

Surfaces assembly would be done at room or lower temperature, so that the hybridization probes do not go off the surface. Detaching probes from the tissue section and attaching to the SGS surface would be regulated by the temperature. First, the temperature of the sandwich would be raised up to denaturize the hybridization probes. Then the temperature would be decreased to allow the common regions of hybridization probes anneal to the oligos immobilized on the SGS surface. In these conditions hybridization probes may hybridize back to the transcripts in tissue sections. However transcripts in the tissue section are few in comparison to oligos on the target surface, so probability to hybridize to the target surface is much higher than back to the tissue section.

The time of denaturation would be selected to allow enough probes to denature and move into solution between the surfaces. The time of hybridization would be adjusted so that enough but not too many probes are transferred to provide a necessary density of sequencing templates and so that probes do not diffuse too far away.

Before separating the surfaces, the temperature would be decreased close to 0°C. At low temperature hybridization speed becomes low, which prevents attaching of probes to the wrong places on SGS surface when sandwich is disturbed. Washing of the unhybridized probes from the SGS flowcell surface would be also performed at low temperature.

Amplification of the transferred probes on the SGS flowcell surface and further sequencing would be performed according to the known sequencing procedures.

SGS would determine two parameters for each probe: (i) its partial or complete nucleotide sequence and (ii) position on the slide surface. Nucleotide sequence will identify which particular transcript was a target for a probe. Position of a probe on a flowcell will be set into correspondence with the position on the tissue section.

### Example 3: Analysis of transcripts in tissue sections by single molecules sequencing transfer of transcripts distribution pattern to the pattern of sequencing templates.

An alternative to SGS analysis as shown in example 2 is described.

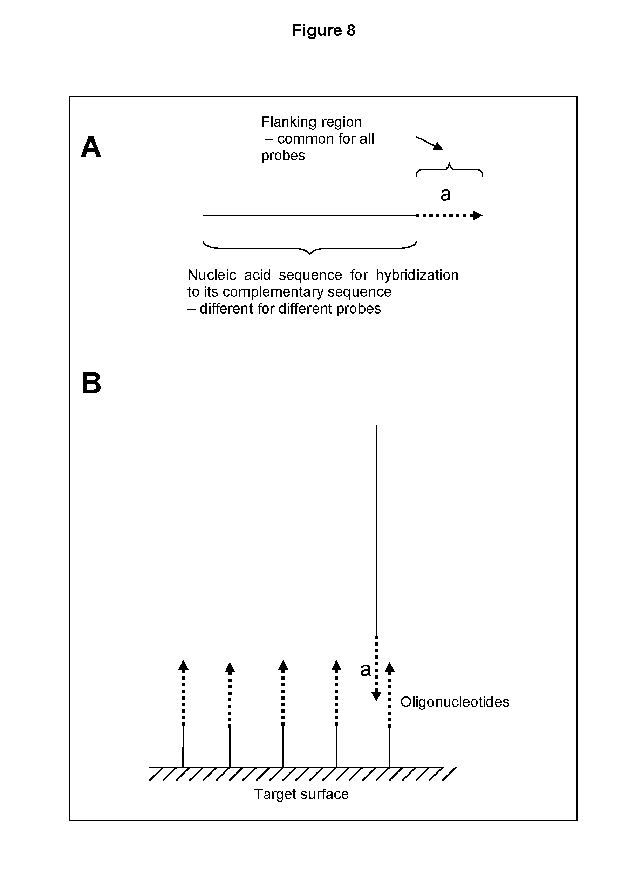

The procedure looks the same as described in example 2, with the difference in sequencing approach: molecules transferred from the tissue section are sequenced directly by single-molecule sequencing approach, where transferred molecules are sequenced directly on the target surface with capturing oligonucleotides initializing the primer extension. Since no amplification on the target surface is required, only one type of oligonucleotides can be present on the sequencing surface for capturing sequencing templates by hybridization. The hybridization probes may have a structure as shown in Fig.8.

This approach may be realized using single-molecule sequencing approach like for example that of Helicos. Single molecules sequencing allows for a higher density of sequencing templates.

### Example 4: Analysis of transcripts in tissue sections by ex situ hybridization.

A further alternative to SGS analysis as shown in example 2 is described.

The procedure looks the same as described in Application Example 2 till amplification of the transferred nucleic acid molecules on the target surface and removing of one strand. Then instead of sequencing, target surface is used for hybridisation with probes of interest. So, this is basically *in situ* hybridization but with targets transferred to another surface and amplified.

In situ amplification results in ∼1000 copies of transferred molecule. This allows increasing hybridization signal and thus sensitivity of transcripts analysis.

Another advantage of this approach is that it makes possible to use same replica for several hybridizations with different probes without increase of background. Target molecules are covalently attached to the surface, so it is possible to use stringent conditions to wash off probes from previous hybridization. This increases the throughput of analysis in comparison to in situ hybridization.

### Example 5a: Marking positions of transcripts in tissue section by nucleic acid codes using a coding surface and subsequent analysis by SGS sequencing

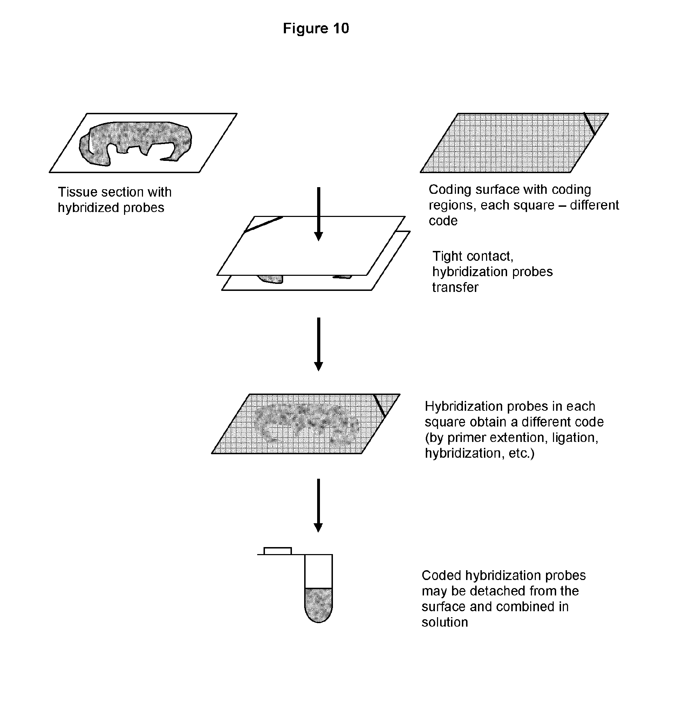

The general scheme of the method is shown in Fig.10. This method allows to transfer transcripts (or corresponding to transcripts hybridization probes, primer extension products) from tissue section into solution preserving information about the distribution pattern. Molecules in the solution may be further processed according to standard sequencing protocols for sample preparation. Loading of sequencing flowcell would be performed as for standard sequencing library, so loading density will be even over the flowcell surface and adjustable. Having sequencing templates in the solution would also allow to use any SGS platform and thus be independent from the SGS surface.

The possible procedure of the experiment is:

preparation of special hybridization probes suitable for
hybridization to the tissue section, hybridization to the coding
surface and having flanking region necessary for SGS

in situ hybridization, washing off the unhybridized probes

transfer of hybridized probes to the coding surface with
prearranged coding regions

coding of transferred hybridization probes by primer
extension reaction

combining coded probes in the solution

clonal amplification (on surface bridge amplification  or e-
PCR, depending on the selected SGS platform);

analysis of sequencing results: identification of transcripts
and their relative positions in tissue section

Hybridization probes would have the structure as shown on figure 9A. Middle parts of probes are for hybridization to transcripts in tissue section. Flanking regions are common for all probes and are required for hybridization to the coding surface (hybr. region) and sequencing on the SGS flowcell surface (seq. region 1).

To prevent unspecific hybridization of common parts of the hybridization probes in tissue section it is possible to reversibly block them with complementary oligonucleotides. These oligonucleotides should be removed before transfer of hybridization probes to the coding surface.

Coding surface is covered with covalently attached coding oligonucleotides. Structure of coding oligonucleotides is shown on Fig.9B. The 3' part, which is complementary to the hybridization region of the hybridization probes, is followed by code region. 5' part is required for further sequencing on the SGS flowcell (seq. region 2). Coding oligo may be detached from the surface using a cleavage site. Cleavage site may be organised for example by a chemically, thermally or enzymatically destroyable nucleotide.

Coding surface consists of coding regions, in each region coding oligos have a different code part. The more precise localisation of transcripts is required, the smaller coding regions should be used. The more coding regions should be on the surface - the longer code region is required to have a unique code in each region.

Hybridized probes would be transferred to a coding surface as described in the example 2. Attachment to the coding surface would be realized by hybridisation of the hybridization region of the hybridization probes to the complementary 3' part of the oligos on the coding surface (Fig. 9B). The result of the transfer would be a coding surface with hybridization probes attached to it in a mirror-distribution relative to the distribution of corresponding transcripts in tissues section.

Transferred hybridization probes would be coded using primer extension reaction (Fig. 9C): depending on the coding region, hybridization probe will be extended with a certain code sequence. Primer extension mix would contain nucleotides and polymerase in an appropriate buffer. Mix would be pipetted over the surface using for example HybriWell chambers from Grace Biolabs. It is important that during primer extension reaction, hybridization probes do not go off their locations. Extension should therefore be performed at temperatures below annealing temperature of hybridization region.

The result of the extension would be double-stranded molecules, in which both strands have flanking regions required for sequencing and code regions, required for revealing molecules position (Fig. 9C). Coded molecules can be removed from the slide and combined in the solution. This may be performed in two ways.

Variant 1. Coding surface would be rinsed in high-temperature (∼95°C) solution. At high temperature, duplexes will be denatured and non-covalently attached strands will go into solution. Also high temperature would inactivate the enzyme used for primer extension, so that no primer extension would be possible in the solution (which may cause chimeric molecules formation). Single-stranded sequencing templates have common flanking regions required for SGS and may be further amplified or used directly for clonal amplification.

Variant 2. Duplexes would be removed from the coding surface after destroying of the cleavable group. Together with duplexes, non-extended coding oligos will also be removed from the coding surface, and may cause extension in solution, which may lead to wrong coding and formation of chimeric molecules. It is therefore necessary to pay attention that polymerase present in primer extention mix is washed away from the surface or inactivated prior to combining the duplexes in solution. Double-stranded sequencing templates (Fig. 9D) may be further amplified or used directly for clonal amplification.

Further stages - amplification of the molecules, clonal amplification and sequencing would be performed according to the known SGS procedures (SOLiD platform from ABI; GA and HiSeq from Illumina).

SGS would determine two sequences for each sequencing template: (i) partial or complete transcript-specific sequence and (ii) sequence of the code. Code sequence will be set into correspondence with the distribution scheme of position coding primers on the tissue section slide, and reveal the initial position of the transcript in the tissue section.

### Example 5b:Marking positions of nucleic acids in tissue section with a sequenced SOLiD flowcell as a coding surface and subsequent analysis by Second Generation Sequencing (SGS)

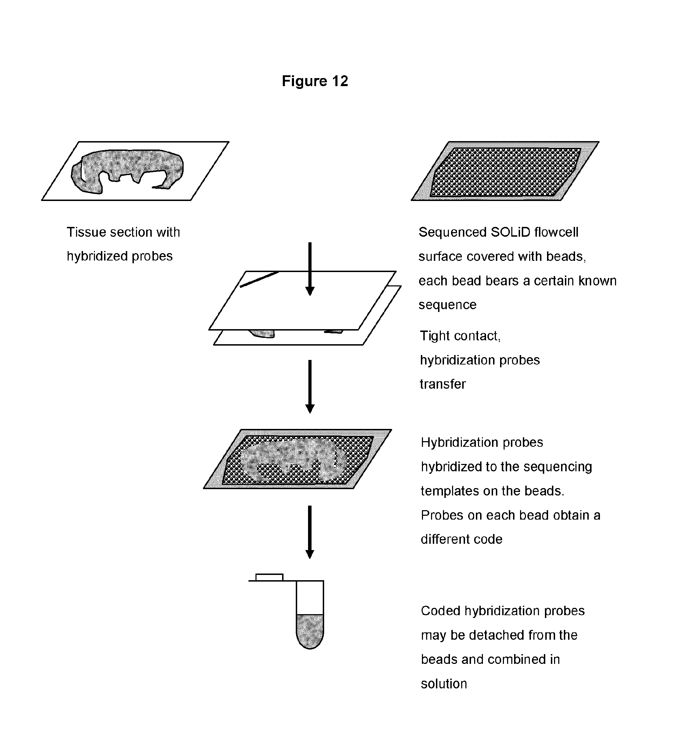

An already sequenced SOLiD flowcell is used as the coding surface. Clonally amplified sequencing templates are attached to the beads. After sequencing, position of each bead and sequence of molecules attached to it are known. Thus, sequences may serve as codes for hybridization probes transferred from tissue section slide (Fig.12).

Hybridization probes would have the structure as shown on Fig.11. Middle parts of probes are for hybridization to transcripts in tissue section. Flanking regions are common for all probes and are required for hybridization to the coding surface (hybridization region) and sequencing on the Illumina platform (illumination region 1). Hybridization region may hybridize to the common 3' region (P2) of SOLiD sequencing templates.

To prevent unspecific hybridization of common parts of the hybridization probes in tissue section it is possible to reversibly block them with complementary oligonucleotides. These oligonucleotides should be removed before transfer of hybridization probes to the coding surface.

Coding surface is a sequenced SOLiD flowcell: glass slide covered with beads. Each bead is a different code region. Unique middle parts of sequencing templates serve as codes (Fig. 11 B).

Hybridized probes would be transferred to the coding surface as described in the example 2. Attachment to the sequencing templates would be realized by hybridization of the hybridization region of the hybridization probes to the complementary P2 regions (Fig. 11 C). The result of the transfer would be beads with hybridization probes attached to them.

Transferred hybridization probes would be coded using primer extension reaction (Fig. 11 D): depending on the bead to which it is attached, hybridization probe will be extended with a certain code sequence. Primer extension mix would contain nucleotides and polymerase in an appropriate buffer. Mix would be pipetted over the surface using for example HybriWell chambers from Grace Biolabs. It is important that during primer extension reaction, hybridization probes do not go off their locations. Extension should therefore be performed at temperatures below annealing temperature of hybridization region. Sequencing templates would not be extended because in the course of the SOLiD sequencing protocol they are 3' end blocked.

The result of the extension would be a hybridization probe to which the sequence of a SOLiD sequencing template is added, and which has a P1 sequence on 3'end (Fig. 11E). Coded molecules may be washed off the beads in denaturizing conditions and combined in solution. Single stranded coded molecules would be amplified to introduce illumination region 2 next to P1 part of the molecule.

Result of amplification would be double-stranded molecules flanked with Illumina-platform specific illumination regions 1 and 2 (Fig.11F), which may be further amplified or used directly for clonal amplification and sequencing on the Illumina platform.

Illumina sequencing would determine two sequences for each sequencing template: (i) partial or complete transcript-specific sequence and (ii) sequence of the code. Code sequences will reveal the position of corresponding beads on the SOLiD flowcell and thus the position of original transcripts in the tissue section.

### Example 6: Analysis of a panel of samples with 2D distributed nucleic acid molecules.

In the previous examples situations were described where the aim was to reveal position of the nucleic acid molecules distributed within tissue section.

For analysis of a panel of samples with 2D distributed nucleic acid molecules (e.g. cell arrays, tissue arrays) it may be necessary to reveal from which sample nucleic acid molecules originate. Procedures described in examples 2-5, work for these applications, too. If coding is used to mark nucleic acid molecules from a single sample, size of coding regions on the coding surface may be comparable to the size of a sample.

### Example 7: Using surfaces covered with oligonucleotides for selective replication.

Replication method using holding nucleic acid molecules on the target surface by hybridization is highly selective. From a mix of nucleic acid molecules transferred from a sample with 2D distributed nucleic acid molecules to the target surface, only those will be replicated, which have a sequence complementary to the capturing oligonucleotides.

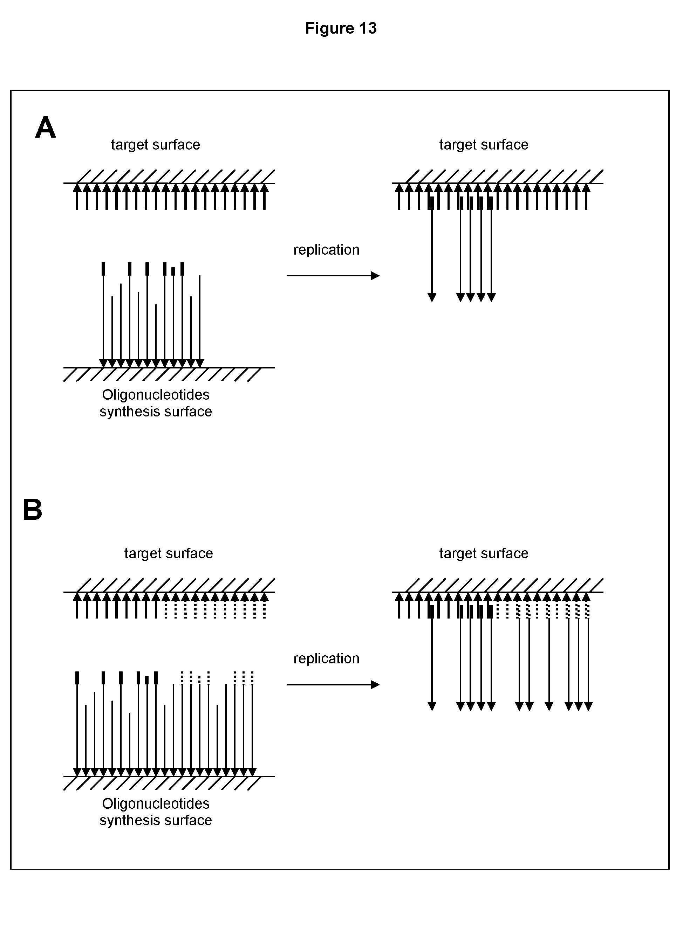

Selectivity may be used for example for selection of full-length oligonucleotides after on-surface synthesis. Oligonucleotides synthesis is performed from 3' to 5' end. For selection of full-length oligos it is possible to use 5' sequences. In the Fig.13A all synthesized oligonucleotides should have the same 5' region. Oligonucleotides complementary to this region are attached to the target surface. During replication of synthesized oligos to the target surface, only full length oligonucleotides get captured.

To create an array of full-length oligonucleotides another approach may be used, involving a coding surface (Fig.13B). Here oligonucleotides synthesized in each array feature have a different 5' region. Capturing oligonucleotides complementary to these 5' regions are synthesized on another array. Features are located in such a way that complementary sequences during replication are opposite to each other.

Such approach is diffusion safe, because oligonucleotides diffused to a neighbor feature would not hybridize to the target surface.

## Claims

1. Using of hybridization of nucleic acids for creating replicas of nucleic acids molecules or molecular complexes containing nucleic acid molecules located either on a surface or within a layer, wherein said creating replicas is obtaining on a target surface the relative distribution of nucleic acid molecules or molecular complexes containing nucleic acid molecules resembling the original distribution.

2. Replication method, comprising the following steps:
a) providing sample with nucleic acid molecules located either on a surface or within a layer;
b) providing target surface with nucleic acid molecules, capable to hybridization-based binding to nucleic acid molecules from (a);
c) (option 1) if nucleic acid molecules are not attached to the sample, providing conditions to minimize shift of molecules from the original positions;
d) (option 2) if nucleic acid molecules are attached to the sample, providing conditions for gradual releasing of nucleic acid molecules;
e) assembling sample with nucleic acid molecules from (a) against the target surface from (b) with solution in between, such that nucleic acid molecules from the sample can reach target surface by diffusion through solution;
f) (option 3) if nucleic acid molecules are attached to the sample, providing conditions for releasing nucleic acid molecules from the original positions in the sample,
g) providing conditions for diffusion of nucleic acid molecules from the sample to the target surface and hybridization-based binding of nucleic acid molecules from the sample to the nucleic acid molecules on the target surface;
h) (optional) providing conditions for slowing down the formation of new hybrids of nucleic acid molecules;
i) disassembling the sample (a) and target surface (b).

3. Replication method according to claim 2, wherein in step h) slowing down formation of new hybrids of nucleic acid molecules is done by decreasing the temperature of the assembly.

4. Replication method according to claim 2, wherein said nucleic acid molecules located on a surface are distributed on a nucleic acid array or protein array, and wherein said nucleic acid molecules distributed within a layer are distributed in a gel layer, in tissue section, in cell or tissue array or in block of tissue.

5. Replication method according to claim 2 wherein in step g) said hybridization-based binding occurs through adapter oligonucleotides which are complementary both to the nucleic acid molecules from the sample and to the nucleic acid molecules on the target surface.

6. Replication method according to claim 2, wherein in step c) said conditions to minimize shift of molecules from the original positions is decrease of temperature.

7. Replication method according to claim 2, wherein in step d) nucleic acid molecules in the sample are held on the original positions by chemical- or enzyme- sensitive binding and said gradual releasing of nucleic acid molecules from the sample is provided by the cleavage agent which acts slow enough to ignore those molecules which change the position before assembling sample against the target surface in step e).

8. Replication method according to claim 7 wherein said conditions for gradual releasing of nucleic acid molecules from the sample by the cleavage agent are provided by decreasing concentration of the said agent or by providing reaction conditions decreasing the activity of the said agent.

9. Replication method according to claim 2, wherein in step f) said condition for releasing the nucleic acid molecules from the original positions in the sample is increasing of the temperature.

10. Replication method according to claim 9, wherein the nucleic acid molecules are held on the original positions in the sample by temperature-sensitive binding or the solution in step e) comprises a thermoactivated cleavage agent.

11. Replication method according to claim 10 wherein said temperature sensitive binding is by hybridization or through thermolabile covalent bonds, abasic site or formaldehyde linkages, and wherein said thermoactivated cleavage agent is an enzyme or chemical reagent.

12. Replication method according to claim 2, wherein in step f) said condition for releasing the nucleic acid molecules from the original positions in the sample is changing the solution between the sample and the target surface.

13. Replication method according to claims 12 wherein the nucleic acid molecules are held on the original positions in the sample by hybridization and said new solution destabilizes hybridization by changing pH or ionic strength of the solution or by decreasing the melting temperature of the duplex like formamide, or the nucleic acid molecules are held on the original positions in the sample by chemical- or enzyme- sensitive binding and said new solution contains a cleavage agent, and wherein either the sample or the target surface or both are permeable for the said solution and during changing of the solution the assembly remains intact.

14. Replication method according to claim 2, wherein in step f) said condition for releasing the nucleic acid molecules from the original positions in the sample is light.

15. Replication method according to claim 14 wherein the nucleic acid molecules are held on the original positions in the sample by photocleavable binding and wherein either the sample or the target surface are transparent for the light with required wavelength.

16. Performing enzymatic reactions with replicas on the target surface according to claim 1, wherein said enzymatic reactions include primer extension, ligation, rolling circle amplification, in situ PCR amplification, bridge PCR amplification, sequencing, restriction.

17. Replication method according to claims 2-16 where nucleic acid molecules in the sample or nucleic acid molecules on the target surface contain known sequences, which (optionally) get inserted in the nucleic acid molecules from the target surface or nucleic acid molecules from the sample by primer extension or ligation reactions and said known sequences are further used for analysis of replicas, wherein said analysis may be performed on the target surface or in solution.

18. Replication method according to claim 17 where the said known sequences serve to distinguish the samples, target surfaces, replication experiments, wherein said known sequences are different between the samples, target surfaces, replication experiments or to determine the position of nucleic acid molecules on the target surface or in the sample and wherein said known sequences are different in different regions of the sample or of the target.
